# EUROPEAN PATENT APPLICATION

(11) **EP 4 599 689 A1**
(43) Date of publication of application: **13.08.2025**
(21) Application number: 23874833.9
(22) Date of filing: 02.10.2023
(51) Int. Cl.: A23K 20/158, A23K 10/30, A23K 20/105, A23K 50/10

(54) **RUMINANT METHANE PRODUCTION INHIBITOR**

(30) Priority: 03.10.2022 JP 2022159773
(71) Applicant: SDS BIOTECH K.K., Tokyo 101-0022 (JP)
(72) Inventor: HIKITA, Chie, Tokyo 101-0022 (JP); INDO, Yuki, Tsukuba-shi, Ibaraki 300-2646 (JP); KANEDA, Koichi, Tokyo 100-8321 (JP); ITO, Shinji, Tokyo 100-8321 (JP)
(74) Representative: Vossius & Partner Patentanwälte Rechtsanwälte mbB
(86) International application number: PCT/JP2023/035963
(87) International publication number: WO 2024/075706

(57) **Abstract**

An agent for inhibiting methane production for ruminants, containing as active components: cashew nut shell liquid, heated cashew nut shell liquid, an anacardic acid, a cardanol, and/or a cardol; and an organic acid, in which the mass ratio of the amount of the cashew nut shell liquid, heated cashew nut shell liquid, anacardic acid, cardanol, and/or cardol, to the amount the organic acid is from 1:5 to 1:20.

## Description

### TECHNICAL FIELD

The present invention relates to agents and feed for inhibiting methane production in ruminants such as cattle, and methods of inhibiting methane production in ruminants using the same.

### BACKGROUND ART

Ruminants such as cattle and sheep have rumen bacteria to digest and ferment their feed in their rumen and utilize the fermentation products. However, methane production by methanogens in rumen decreases the energy efficiency from the feed. Furthermore, methane is a greenhouse gas that affects global warming. Thus, it is important to reduce methane production in rumen of ruminants.

Methanogens in rumen reduce carbon dioxide with hydrogen to produce methane. It is said that the contribution of methane to global warming is second highest after carbon dioxide, and methane emitted from ruminants accounts for 15 to 20% of the total methane emission.

Cashew nut shell liquid is known to have effects on ruminants, including rumen fermentation improving effects such as methane production-inhibiting effects (Patent Document 1: WO2008/149992). In response to recent global awareness of the need to prevent global warming, there have been required further improvements.

On the other hand, Animal Feed Science and Technology, Volumes 166-167, 23 June 2011, Pages 282-290 (Non-Patent Document 1) and Journal of Dairy Science, Volume 92, Issue 7, July 2009, Pages 3258-3264 (Non-Patent Document 2) disclose inhibition of methane production by malic acid. However, it uses malic acid at very high concentrations and thus is unfeasible.

In addition, WO2011/152532 (Patent Document 2) and WO2012/060451 (Patent Document 3) have disclosed the addition of acids such as organic acids to prevent decomposition of and improve the stability of anacardic acid in cashew nut shell liquid-containing compositions. However, the organic acids have been only added in trace amounts relative to the cashew nut shell liquid, and of course, there has been no suggestion on inhibition of methane production.

### [Prior Art Documents]

### [Patent Document]

[Patent Document 1] WO2008/149992
[Patent Document 2] WO2011/152532
[Patent Document 3] WO2012/060451

### [Non-Patent Document]

[Non-Patent Document 1] Animal Feed Science and Technology, Volumes 166-167, 23 June 2011, Pages 282-290
[Non-Patent Document 2] Journal of Dairy Science, Volume 92, Issue 7, July 2009, Pages 3258-3264

### SUMMARY OF THE INVENTION

### Problems to be solved by the invention

The present invention aims to provide a material for efficiently inhibiting the methane production while maintaining the energy production and metabolism in ruminants.

### Means to solve the problems

In order to solve the above problem, the present inventors have intensively studied to find that administration of cashew nut shell liquid (hereinafter may also be referred to as CNSL) and the like and an organic acid to a ruminant in a specific ratio has an effect of efficiently inhibiting methane production in the ruminant while maintaining the production of acetic acid that is a main material for the synthesis of energy and lipid in ruminants. Based on such findings, the present inventors have completed the present invention.

Accordingly, the present invention is as follows:
[1] An agent for inhibiting methane production for ruminants, comprising as active components:
   cashew nut shell liquid, heated cashew nut shell liquid, an anacardic acid, a cardanol, and/or a cardol; and
   an organic acid,
   wherein the mass ratio of the amount of the cashew nut shell liquid, heated cashew nut shell liquid, anacardic acid, cardanol, and/or cardol, to the amount the organic acid is from 1:5 to 1:20 (alternatively 1:10 to 1:20);
[2] The agent for inhibiting methane production of [1], wherein the organic acid is a C₃₋₁₂ carboxylic acid;
[3] The agent for inhibiting methane production of [1], wherein the organic acid is a C₃₋₁₀ hydroxycarboxylic acid;
[4] The agent for inhibiting methane production of [1], wherein the organic acid is citric acid or malic acid;
[5] The agent for inhibiting methane production of any one of [1] to [3], wherein the ruminant is cattle;
[6] A food for inhibiting methane production for ruminants, comprising the agent for inhibiting methane production of any one of [1] to [5];
[7] A method of inhibiting methane production in a ruminant, comprising administering cashew nut shell liquid, heated cashew nut shell liquid, an anacardic acid, a cardanol, and/or a cardol, and an organic acid to the ruminant,
   wherein the mass ratio of the daily dose per ruminant of the cashew nut shell liquid, heated cashew nut shell liquid, anacardic acid, cardanol, and/or cardol to the daily dose per ruminant of the organic acid is from 1:5 to 1:20 (alternatively 1:10 to 1:20);
[8] The method of [7], wherein the organic acid is a C₃₋₁₂ carboxylic acid;
[9] The method of [7], wherein the organic acid is a C₃₋₁₀ hydroxycarboxylic acid;
[10] The method of [7], wherein the organic acid is citric acid or malic acid;
[11] The method of any one of [7] to [10], wherein the ruminant is cattle;
[12] The method of any one of [7] to [11], wherein the cashew nut shell liquid, heated cashew nut shell liquid, anacardic acid, cardanol, and/or cardol, and the organic acid are pre-mixed and administered;
[13] The method of any one of [7] to [11], wherein the cashew nut shell liquid, heated cashew nut shell liquid, anacardic acid, cardanol, and/or cardol, and the organic acid are separately administered.
[14] Use of cashew nut shell liquid, heated cashew nut shell liquid, an anacardic acid, a cardanol, and/or a cardol; and an organic acid in manufacturing of an agent for inhibiting methane production for ruminants,
   wherein the mass ratio of the amount of the cashew nut shell liquid, heated cashew nut shell liquid, anacardic acid, cardanol, and/or cardol, to the amount the organic acid is from 1:5 to 1:20 (alternatively 1:10 to 1:20);
[15] The use of [14], wherein the organic acid is a C₃₋₁₂ carboxylic acid;
[16] The use of [14], wherein the organic acid is a C₃₋₁₀ hydroxycarboxylic acid;
[17] The use of [14], wherein the organic acid is citric acid or malic acid;
[18] The use of any one of [14] to [17], wherein the ruminant is cattle;
[19] Non-therapeutic use of cashew nut shell liquid, heated cashew nut shell liquid, an anacardic acid, a cardanol, and/or a cardol; and an organic acid for inhibiting methane production for ruminants,
   wherein the mass ratio of the amount of the cashew nut shell liquid, heated cashew nut shell liquid, anacardic acid, cardanol, and/or cardol, to the amount the organic acid is from 1:5 to 1:20 (alternatively 1:10 to 1:20);
[20] The use of [19], wherein the organic acid is a C₃₋₁₂ carboxylic acid;
[21] The use of [19], wherein the organic acid is a C₃₋₁₀ hydroxycarboxylic acid;
[22] The use of [19], wherein the organic acid is citric acid or malic acid;
[23] The use of any one of [19] to [22], wherein the ruminant is cattle.

### EFFECT OF THE INVENTION

According to the present invention, administration of cashew nut shell liquid, heated cashew nut shell liquid, an anacardic acid, a cardanol, and/or a cardol, and an organic acid to a ruminant in a specific ratio can efficiently inhibit methane production in the rumen while maintaining the production of acetic acid that is a main material for the synthesis of energy and lipid in ruminants. This can contribute to the prevention of global warming in the livestock field while maintaining the growth and productivity of livestock. In addition, there is the advantage that cashew nut shell liquid can act at low doses and is highly safe.

### DETAILED DESCRIPTION OF THE INVENTION

The agent for inhibiting methane production of the present invention comprises as active components: cashew nut shell liquid (CNSL), heated cashew nut shell liquid, an anacardic acid, a cardanol, and/or a cardol (hereinafter may be referred to as "cashew nut shell liquid and/or the like"); and an organic acid, wherein the mass ratio of the amount of the cashew nut shell liquid, heated cashew nut shell liquid, anacardic acid, cardanol, and/or cardol, to the amount the organic acid is from 1:5 to 1:20 (alternatively 1:10 to 1:20).

Here, the amount of the cashew nut shell liquid, heated cashew nut shell liquid, anacardic acid, cardanol, and/or cardol, when including two or more thereof, is the total amount. The amount of the organic acid, when including two or more organic acids, is also the total amount.

Cashew nut shell liquid, when described in distinction from heated cashew nut shell liquid, means unheated cashew nut shell liquid.

Among cashew nut shell liquid (unheated), heated cashew nut shell liquid, anacardic acid, and cardanol, cashew nut shell liquid (unheated) or anacardic acid is preferable, and cashew nut shell liquid (unheated) is particularly preferable.

Cashew nut shell liquid is an oily liquid contained in nutshells of cashew nut trees (Anacardium occidentale L.). Cashew nut shell liquid contains anacardic acids, cardanols, and cardols as its components. Anacardic acids are converted into cardanols by heat treatment. Cashew nut shell liquid only containing cardanols and cardols after heat treatment (heated cashew nut shell liquid) may also be used.

Cashew nut shell liquid (unheated) extracted by cold-pressing cashew nut shells as described in J. Agric. Food Chem. 2001, 49, 2548-2551 contains 55 to 80% by mass of anacardic acids, 5 to 20% by mass of cardanols, and 5 to 30% by mass of cardols.

The heated cashew nut shell liquid obtained by heating unheated cashew nut shell liquid (e.g., at 70°C or higher, preferably at 130°C or higher), in which anacardic acids that are main components of the unheated cashew nut shell liquid have been decarboxylated and converted into cardanols, contains 0 to 10% by mass of anacardic acids, 55 to 80% by mass of cardanols, and 5 to 30% by mass of cardols.

Cashew nut shell liquid can be obtained as a vegetable oil extracted by cold-pressing from cashew nut shells. Cashew nut shell liquid can also be obtained by dry distillation or solvent extraction of cashew nut shells. Cashew nut shell liquid can also be obtained, for example, by a method described in JP H8-231410 A. A commercially available product of cashew nut shell liquid can also be used.

Instead of cashew nut shell liquid, the agent for inhibiting methane production of the present invention may comprise one or more of anacardic acids, cardanols, and cardols.

The anacardic acids include naturally-occurring anacardic acids, synthetic anacardic acids, and derivatives thereof. A commercially available anacardic acid may also be used. Anacardic acids can be obtained, as described in JP H8-231410 A, by extracting a cashew nut oil from cashew nut shells with an organic solvent, and subjecting the obtained cashew nut oil to, for example, silica gel column chromatography for elution with a varying ratio of n-hexane, ethyl acetate, and acetic acid in the mixed solvent (e.g., JP H3-240721 A, JP H3-240716 A).

The cardanols include naturally-occurring cardanols, synthetic cardanols, and derivatives thereof. The cardanols used in the present invention can be obtained by decarboxylating of anacardic acids that are the main components of cashew nut shell liquid.

The cardols include naturally-occurring cardols, synthetic cardols, and derivatives thereof. The cardols used in the present invention can be obtained by purifying from cashew nut shell liquid.

The amount of the cashew nut shell liquid, heated cashew nut shell liquid, anacardic acid, cardanol, and/or cardol (which is the total amount, when two or more thereof are contained) in the agent for inhibiting methane production of the present invention is, for example, 1% by mass to 20% by mass relative to the total amount of the inhibitor.

The type of the organic acid is not particularly limited, and is preferably a carboxylic acid. Examples of the carboxylic acid include monocarboxylic acids, dicarboxylic acids, and tricarboxylic acids, including aliphatic carboxylic acids, aliphatic hydroxycarboxylic acids, aromatic carboxylic acids, aromatic hydroxycarboxylic acids, and amino acids. The carbon number of the organic acid, including the carbon of the carboxyl group, is preferably 3 or more, and more preferably 4 or more, and is preferably 12 or less, more preferably 10 or less, still more preferably 8 or less, and particularly preferably 7 or less. Specific examples of the organic acid include formic acid, acetic acid, propionic acid, butyric acid, valeric acid, salicylic acid, lactic acid, tartaric acid, citric acid, leucic acid, malic acid, fumaric acid, ricinoleic acid, quinic acid, and shikimic acid, and citric acid, malic acid, or fumaric acid is particularly preferable.

The amount of the organic acid in the agent for inhibiting methane production (which is the total amount, when two or more organic acids are contained) is, for example, 5% by mass to 95% by mass relative to the total amount of the inhibitor.

The methane production-inhibiting effect of the agent for inhibiting methane production of the present invention can be determined, for example, as in Examples described later, by comparing the methane production when CNSL and the like and an organic acid are added to a culture of ruminant rumen fluid in the specific ratio described followed by incubation with the production in a control (without addition of CNSL and the like or an organic acid). CNSL and the like and an organic acid may be directly administered to a ruminant for comparison of methane production. The methane production is preferably reduced by the effect of the CNSL and the like and the organic acid, as compared with the control (without addition of CNSL and the like or an organic acid), to 90% or less, preferably 80% or less, and more preferably 50% or less.

In addition to the cashew nut shell liquid, heated cashew nut shell liquid, anacardic acid, cardanol, and/or cardol, and the organic acid, the agent for inhibiting methane production of the present invention may comprise, for example, excipients and other components that can be used in feeds or pharmaceuticals, such as lactose, saccharose, D-mannitol, α-starch, starch, saccharose, crystalline cellulose, bentonite, silica gel, and light anhydrous silicic acid.

The agent for inhibiting methane production of the present invention may further comprise optional components such as components that are effective in growth promotion of ruminants, nutrition supplemental components, storage stability-enhancing components, and coating components, in addition to the cashew nut shell liquid, heated cashew nut shell liquid, anacardic acid, cardanol, and/or cardol, and the organic acid. Examples of such optional components include raw materials of feeds, such as bran, alfalfa, and timothy, feed additives, food source materials, food additives, pharmaceutical product materials, and other supplement components used in supplements for animals (hereinafter referred to as "supplements"). Examples thereof include: living bacterial agents such as Enterococcus spp., Bacillus spp., and Bifidobacterium spp.; enzymes such as amylase and lipase; L-ascorbic acid, choline chloride, inositol, and folic acid; minerals such as potassium chloride, magnesium oxide, and phosphates; antioxidants such as ethoxyquin, dibutyl hydroxytoluene, butylated hydroxyanisole, ferulic acid, vitamin C, and vitamin E; fungicides such as calcium propionate; binding agents such as carboxymethyl cellulose (CMC), sodium caseinate, and sodium polyacrylate; emulsifiers such as lecithin, glycerin fatty acid ester, and sorbitan fatty acid ester; coloring agents such as astaxanthin and canthaxanthin; and flavoring agents such as various esters, ethers, and ketones.

The agent for inhibiting methane production of the present invention may comprise an oil absorption agent such as magnesium oxide, stearate, talc, zeolite, diatom earth, and silica, and the oil absorption agent is preferably particulate. The oil absorption agent preferably absorbs 50 to 300 g of an oil per 100 g of the oil absorption agent. Since particles having particle diameters exceeding 300 µm are coarse and will be separated, those having particle diameters of 2 to 300 µm are preferable.

In one embodiment, a preferred mass ratio of the oil absorption agent, to the cashew nut shell liquid, heated cashew nut shell liquid, anacardic acid, cardanol, and/or cardol, and the organic acid in the agent for inhibiting methane production of the present invention is 100:20 to 100: 180.

The dosage form of the agent for inhibiting methane production of the present invention is not particularly limited, and may be any form, for example, solution, powder, solid, tablet, capsule, emulsion, pellet, or coating, and is preferably solution, powder, capsule, pellet, or tablet.

As solutions, cashew nut shell liquid, heated cashew nut shell liquid, anacardic acid, cardanol, and/or cardol may be added, directly or after being solved in solvents such as ethanol, with an organic acid, and the solutions can be used with further the addition of excipients or optional components as described above. Powders, capsules, pellets, or tablets as described below may be suspended in the solutions.

As powders, cashew nut shell liquid, heated cashew nut shell liquid, anacardic acid, cardanol, and/or cardol, and an organic acid may be powdered with the addition of excipients as described above.

As capsules, cashew nut shell liquid, heated cashew nut shell liquid, anacardic acid, cardanol, and/or cardol, and an organic acid may be capsulated directly or with the addition of excipients or optional components as described above.

As pellets, cashew nut shell liquid, heated cashew nut shell liquid, anacardic acid, cardanol, and/or cardol, and an organic acid may be mixed with excipients as described above, granulated, and pelletized.

As tablets, cashew nut shell liquid, heated cashew nut shell liquid, anacardic acid, cardanol, and/or cardol, and an organic acid may be mixed with excipients as described above, granulated, and made into tablets.

In cases where oil absorption agents such as silica are contained, formulation as powders, tablets, or pellets is preferable.

As described above, the agent for inhibiting methane production of the present invention can be manufactured by mixing cashew nut shell liquid, heated cashew nut shell liquid, an anacardic acid, a cardanol and/or a cardol with an organic acid, further mixed as needed with excipients or optional components, and formulated. Depending on the form of the agent, the agent for inhibiting methane production of the present invention can be obtained by mixing the crushed or ground cashew nut shells as described above or intact cashew nut shells without any treatment can be mixed with an organic acid and other optional components.

The cashew nut shell liquid, heated cashew nut shell liquid, anacardic acid, cardanol, and/or cardol and the organic acid are preferably mixed with animal feed for administration.

Thus, the present invention provides feed for inhibition of methane production in ruminants, comprising cashew nut shell liquid, heated cashew nut shell liquid, an anacardic acid, cardanol, and/or cardol, and an organic acid as active components.

The feed of the present invention comprises cashew nut shell liquid, heated cashew nut shell liquid, an anacardic acid, a cardanol, and/or a cardol, and an organic acid.

The total amount of the cashew nut shell liquid, heated cashew nut shell liquid, anacardic acid, cardanol, and/or cardol (which is the total amount, when two or more are contained) in the feed of the present invention is not particularly limited as long as it is such an amount that the mass ratio of the amount of the added cashew nut shell liquid, heated cashew nut shell liquid, anacardic acid, cardanol, and/or cardol, to the amount of the organic acid is 1:5 to 1:20 (alternatively 1:10 to 1:20).

The feed of the present invention can be manufactured by adding cashew nut shell liquid, heated cashew nut shell liquid, an anacardic acid, a cardanol, and/or a cardol, and an organic acid, or the agent for inhibiting methane production of the present invention comprising them to a feed component, and mixing them.

In this case, and when a powder or solid agent is used, the agent may be made into a liquid or gel form using a liquid carrier in order to facilitate the mixing. In this case, liquid with fluidity, such as water, a vegetable oil, a liquid animal oil, a mineral oil, a synthetic oil, or an aqueous polymer compound, can be used as the liquid carrier. To maintain the homogeneity of the cashew nut shell liquid, heated cashew nut shell liquid, anacardic acid, cardanol, and/or cardol, and the organic acid in the feed, aqueous polysaccharides such as alginic acid, sodium alginate, xanthan gum, carboxymethylcellulose, a starch, sodium caseinate, gum arabic, guar gum, tamarind seed polysaccharides may also be preferably added.

The type, ratio, and the like of the feed component to be mixed with the agent of the present invention in the feed of the present invention are not particularly limited, as long as it is feed that has been conventionally supplied to the animals. For example, corn grain, corn flour, milo, bran, soybean cake, oat, wheat flour short, wheat coarse flour, alfalfa, timothy, clover, defatted rice bran, white fish meal, coastal fish meal, yeast, molasses, meat pieces, bone meal, calcium carbonate, calcium phosphate dibasic, yellow grease, vitamins, and minerals can be used for preparation.

The present invention also provides a method of inhibiting methane production in ruminants, comprising a step of administering cashew nut shell liquid, heated cashew nut shell liquid, an anacardic acid, a cardanol, and/or a cardol, and an organic acid to the ruminant, wherein the mass ratio of the daily dose per ruminant of the cashew nut shell liquid, heated cashew nut shell liquid, anacardic acid, cardanol, and/or cardol to the daily dose per ruminant of the organic acid is from 1:5 to 1:20 (alternatively 1:10 to 1:20).

The cashew nut shell liquid, heated cashew nut shell liquid, anacardic acid, cardanol, and/or cardol, and the organic acid may be pre-mixed and administered, such as by administration of a feed containing them, or may be separately administered to ruminants. When administered (fed) separately, the timing of administration is not particularly limited as long as the cashew nut shell liquid, heated cashew nut shell liquid, anacardic acid, cardanol, and/or cardol, and the organic acid are ingested by ruminants and then coexist in the body, especially in the rumen at that timing.

The type of ruminant to be fed with the feed of the present invention is not particularly limited, and examples thereof include cattle, buffalo, goat, sheep, and yak. The species of the cattle include, but not limited to, Holstein, Jersey, Japanese Black, Japanese Shorthorn, and Aberdeen Angus. In addition, the species are not limited to any particular use, for example, for livestock production, such as dairy or beef, or for breeding. Examples of the period for feeding the present agent include, but not limited to, use after 4 weeks of age when the ruminant stomach begins to functionally form.

The dose (which is the total amount, when two or more are contained) of the cashew nut shell liquid, heated cashew nut shell liquid, anacardic acid, cardanol, and/or cardol, as a daily dose per ruminant, can be, for example, 0.5 to 50 g/head/day, preferably 1 to 25 g/head/day, and more preferably 2.5 to 10 g/head/day. The dose (which is the total amount, when two or more are contained) of the organic acid, as a daily dose per ruminant, can be, for example, 1 to 1,000 g/head/day, preferably 5 to 200 g/head/day, and more preferably 12.5 to 150 g/head/day. It is noted that the mass ratio of the daily dose per ruminant of the cashew nut shell liquid, heated cashew nut shell liquid, anacardic acid, cardanol, and/or cardol to the daily dose per ruminant of the organic acid is adjusted to 1:5 to 1:20 (alternatively 1:10 to 1:20).

Two or more administrations may be performed in a single day. The administration may be performed for several days.

### EXAMPLES

The present invention will now be described in detail by referring to Examples. However, the present invention is not limited thereto.

### Example 1

### Evaluation of Cashew Nut Shell Liquid (CNSL), Organic Acid, and Garlic Powder by Methane Production Measurement System Using Rumen Culture

### (1) Sample

Cashew nut shell liquid (CNSL) cold-pressed and extracted from cashew nut shells was obtained. For culture inoculum, rumen fluids (filtrates through quadruple gauzes) collected orally from Holstein milking cows 90 days or longer after calving were used. 4 mL of the rumen fluid and 6 mL of artificial saliva (McDougal's artificial saliva) were added, and the mixture was used for culturing.

### (2) Culturing

The culturing was performed with the concentration of CNSL in the test culture being 100 to 500 mg/L. CNSL was dissolved in ethanol and added to a Hungate tube such that it had a desired concentration in the solution. Similarly, DL-malic acid and fumaric acid were dissolved in ethanol and added such that they had desired concentrations in the solution. To equalize the effect of ethanol, only ethanol was added to a no-addition (control) area. Thereafter, the resulting products were left for several hours to vaporize ethanol, to which 50 mg of corn starch, 75 mg of concentrate feed powder, and 75 mg of timothy hay powder were added as culture substrates. The garlic powder was added in an amount of 0.5% or 1.0% relative to the dry weight of the culture substrates. This corresponds to 100 mg/L or 200 mg/L in terms of concentration in the test culture. To these test tubes, a total of 10 ml of diluted rumen fluid and artificial saliva in the above mixture ratio was added, and the tubes were incubated anaerobically in an incubator (39°C, 17 hours) with butyl rubber caps and plastic screw caps provided while nitrogen gas was blown into the head space. Each culturing was performed in triplicate.

### (3) Analysis

The concentrations of methane and acetic acid in volatile fatty acids were both analyzed by FID gas chromatography.

### (4) Results

### (i) Gas Production and Acetic Acid Production

The gas production and acetic acid production results are shown in Table 1.

Reduction of the methane production and the acetic acid production depending on the CNSL concentration was observed. In cases where 100 mg/L CNSL and 500 to 1,000 mg/L DL-malic acid or 500 mg/L fumaric acid were used in combination, the methane production was significantly reduced as compared to the addition of 100 mg/L CNSL alone, while the acetic acid production did not show a significant reduction. The use of CNSL and garlic powder in combination, of 500 to 1,000 mg/L DL-malic acid alone, and of 100 to 200 mg/L garlic powder did not show significant improvement in the methane reduction rate.

**[Table 1]**

| | methane amount | | acetate amount | |
|---|---|---|---|---|
| | mL/tube(mean±SD) | %reduction vs | g/L(mean±SD) | % vs cont |
| Control | 5.48 ± 0.08 | cont - | 3.39 ± 0.31 | - |
| CNSL 100 mg/L | 5.00 ± 0.27 | ▲9% | 3.50 ± 0.22 | 103% |
| CNSL 200 mg/L | 1.91 ± 0.28 | ▲65% | 2.57 ± 0.31 | 76% |
| CNSL 500 mg/L | 0.30 ± 0.03 | ▲95% | 1.51 ± 0.10 | 44% |
| DL-malic acid 500 mg/L | 6.22 ± 0.93 | 14% | 4.18 ± 0.11 | 123% |
| DL-malic acid 500 mg/L+CNSL 100 mg/L | 4.44 ± 0.05 | ▲19% | 3.60 ± 0.13 | 106% |
| DL-malic acid 1000 mg/L | 5.47 ± 0.01 | ▲0% | 3.76 ± 0.11 | 111% |
| DL-malic acid 1000 mg/L+CNSL 100 mg/L | 2.90 ± 0.80 | ▲47% | 3.50 ± 0.15 | 103% |
| fumaric acid 500 mg/L | 5.94 ± 0.01 | 8% | 3.86 ± 0.12 | 114% |
| fumaric acid 500 mg/L+CNSL 100 mg/L | 2.89 ± 0.34 | ▲47% | 3.23 ± 0.18 | 95% |
| garlic powder 100 mg/L | 5.41 ± 0.25 | ▲1% | 4.11 ± 0.16 | 121% |
| garlic powder 100 mg/L+CNSL 100 mg/L | 5.08 ± 0.08 | ▲7% | 4.00 ± 0.16 | 118% |
| garlic powder 200 mg/L | 5.59 ± 0.06 | 2% | 3.78 ± 0.10 | 111% |
| garlic powder 200 mg/L+CNSL 100 mg/L | 5.15 ± 0.37 | ▲6% | 4.19 ± 0.26 | 124% |

### Example 2

### Evaluation of Cashew Nut Shell Liquid (CNSL), and Various Organic Acids by Methane Production Measurement System Using Rumen Culture

### (1) Sample

Cashew nut shell liquid (CNSL) cold-pressed and extracted from cashew nut shells was obtained. For culture inoculum, rumen fluids (filtrates through quadruple gauzes) collected orally from Holstein milking cows 90 days or longer after calving were used. 4 mL of the rumen fluid and 6 mL of artificial saliva (McDougal's artificial saliva) were added and the mixture was used for culturing.

### (2) Culturing

The culturing was performed with the concentration of CNSL in the test culture being 100 to 500 mg/L. CNSL was dissolved in ethanol and added to a Hungate tube such that it had any concentration in the solution. Similarly, pyruvic acid, citric acid, and succinic acid were dissolved in ethanol and added such that they had desired concentrations in the solution. To equalize the effect of ethanol, only ethanol was added to a no-addition (control) area. Thereafter, the resulting products were left for several hours to vaporize ethanol, to which 50 mg of corn starch, 75 mg of concentrate feed powder, and 75 mg of timothy hay powder were added as culture substrates. To these test tubes, a total of 10 ml of diluted rumen fluid and artificial saliva in the above mixture ratio was added, and the tubes were incubated anaerobically in an incubator (39°C, 17 hours) with butyl rubber caps and plastic screw caps provided while nitrogen gas was blown into the head space. Each culturing was performed in triplicate.

### (3) Analysis

The concentrations of methane and acetic acid in volatile fatty acids were both analyzed by FID gas chromatography.

### (4) Results

### (i) Gas Production and Acetic Acid Production

The gas production and acetic acid production results are shown in Table 2.

Same as Example 1, a reduction of the methane production and the acetic acid production depending on the CNSL concentration was observed. In cases where 100 mg/L CNSL and 500 to 1,000 mg/L citric acid were used in combination, the methane production was significantly reduced as compared to the addition of 100 mg/L CNSL alone, while the acetic acid production showed no reduction. The use of CNSL and pyruvic acid or succinic acid in combination or the addition of each of the organic acids alone at the concentrations did not show significant improvement in the methane reduction rate.

**[Table 2]**

| | methane amount | | acetate amount | |
|---|---|---|---|---|
| | mL/tube(mean±SD) | % reduction vs cont | g/L(mean±SD) | % vs cont |
| Control | 4.77 ± 0.15 | - | 3.70 ± 0.62 | - |
| CNSL 100 mg/L | 3.70 ± 0.63 | ▲22% | 3.53 ± 0.19 | 95% |
| CNSL 200 mg/L | 2.01 ± 1.18 | ▲58% | 2.54 ± 0.04 | 69% |
| CNSL 500 mg/L | 0.24 ± 0.06 | ▲95% | 1.14 ± 0.18 | 31% |
| pyruvic acid 500 mg/L | 5.58 ± 1.26 | 17% | 4.04 ± 0.31 | 109% |
| pyruvic acid 500 mg/L+CNSL 100 mg/L | 4.41 ± 0.63 | ▲7% | 4.00 ± 0.13 | 108% |
| pyruvic acid 1000 mg/L | 5.39 ± 0.04 | 13% | 4.36 ± 0.02 | 118% |
| pyruvic acid 1000 mg/L+CNSL 100 mg/L | 4.45 ± 0.85 | ▲7% | 4.31 ± 0.05 | 116% |
| citric acid 500 mg/L | 5.12 ± 0.67 | 7% | 4.25 ± 0.16 | 115% |
| citric acid 500 mg/L+CNSL 100 mg/L | 3.63 ± 0.59 | ▲24% | 4.22 ± 0.24 | 114% |
| citric acid 1000 mg/L | 3.77 ± 1.64 | ▲21% | 4.42 ± 0.14 | 119% |
| citric acid 1000 mg/L+CNSL 100 mg/L | 2.32 ± 0.97 | ▲51% | 4.41 ± 0.08 | 119% |
| succinic acid 500 mg/L | 5.28 ± 1.76 | 11% | 4.26 ± 0.42 | 115% |
| succinic acid 500 mg/L+CNSL 100 mg/L | 4.60 ± 1.34 | ▲4% | 4.07 ± 0.12 | 110% |
| succinic acid 1000 mg/L | 6.24 ± 0.83 | 31% | 3.91 ± 0.58 | 106% |
| succinic acid 1000 mg/L+CNSL 100 mg/L | 4.22 ± 0.43 | ▲12% | 3.70 ± 0.30 | 100% |

### Example 3

### Evaluation of Cashew Nut Shell Liquid (CNSL), and DL-malic Acid and Citric Acid by Methane Production Measurement System Using Rumen Culture

### (1) Sample

Cashew nut shell liquid (CNSL) cold-pressed and extracted from cashew nut shells was obtained. For culture inoculum, rumen fluids (filtrates through quadruple gauzes) collected orally from Holstein milking cows 90 days or longer after calving were used. 4 mL of the rumen fluid and 6 mL of artificial saliva (McDougal's artificial saliva) were added and the mixture was used for culturing.

### (2) Culturing

The culturing was performed with the concentration of CNSL in the test culture being 100 to 500 mg/L. CNSL was dissolved in ethanol and added to a Hungate tube such that it had any concentration in the solution. Similarly, DL-malic acid and citric acid were dissolved in ethanol and added such that they had required concentrations in the solution. To equalize the effect of ethanol, only ethanol was added to a no-addition (control) area. Thereafter, the resulting products were left for several hours to vaporize ethanol, to which 50 mg of corn starch, 75 mg of concentrate feed powder, and 75 mg of timothy hay powder were added as culture substrates. To these test tubes, a total of 10 mL of diluted rumen fluid and artificial saliva in the above mixture ratio was added, and the tubes were incubated anaerobically in an incubator (39°C, 17 hours) with butyl rubber caps and plastic screw caps provided while nitrogen gas was blown into the head space. Each culturing was performed in triplicate.

### (3) Analysis

The concentrations of methane and acetic acid in volatile fatty acids were both analyzed by FID gas chromatography.

### (4) Results

### (i) Gas Production and Acetic Acid Production

The gas production and acetic acid production results are shown in Table 3.

Same as Examples 1 and 2, a reduction of the methane production and the acetic acid production depending on the CNSL concentration was observed. In cases where 100 mg/L CNSL and 500 to 2,000 mg/L DL-malic acid or 500 to 2,000 mg/L citric acid were used in combination, the methane production was significantly reduced as compared to the addition of 100 mg/L CNSL alone, while the acetic acid production showed no reduction. The use of a mixture of 500 mg/L citric acid and 500 mg/L DL-malic acid showed tendency to be more effective in reducing methane than the addition of each organic acid alone.

**[Table 3]**

| | methane amount | | acetate amount | |
|---|---|---|---|---|
| | mL/tube(mean±SD) | %reduction vs cont | g/L(mean±SD) | % vs cont |
| Control | 6.12 ± 0.08 | | 3.68 ± 0.66 | - |
| CNSL 100 mg/L | 5.08 ± 0.52 | ▲17% | 4.26 ± 0.26 | 116% |
| CNSL 200 mg/L | 1.95 ± 0.16 | ▲68% | 2.57 ± 0.23 | 70% |
| CNSL 500 mg/L | 0.42 ± 0.21 | ▲93% | 1.51 ± 0.17 | 41% |
| DL-malic acid 500 mg/L | 6.13 ± 0.92 | 0% | 4.18 ± 0.07 | 114% |
| DL-malic acid 500 mg/L+CNSL 100 mg/L | 5.54 ± 1.36 | ▲9% | 3.79 ± 0.14 | 103% |
| DL-malic acid 1000 mg/L | 6.13 ± 0.00 | 0% | 3.76 ± 0.27 | 102% |
| DL-malic acid 1000 mg/L+CNSL 100 mg/L | 3.78 ± 0.60 | ▲38% | 3.87 ± 0.14 | 105% |
| DL-malic acid 2000 mg/L | 5.95 ± 0.70 | ▲3% | 3.85 ± 0.38 | 105% |
| DL-malic acid 2000 mg/L+CNSL 100 mg/L | 3.60 ± 0.39 | ▲41% | 3.49 ± 0.34 | 95% |
| citric acid 500 mg/L | 6.53 ± 0.43 | 7% | 3.64 ± 0.70 | 99% |
| citric acid 500 mg/L+CNSL 100 mg/L | 5.46 ± 0.41 | ▲11% | 4.14 ± 0.59 | 112% |
| citric acid 1000 mg/L | 6.41 ± 1.49 | 5% | 3.76 ± 1.10 | 102% |
| citric acid 1000 mg/L+CNSL 100 mg/L | 4.50 ± 0.68 | ▲26% | 4.79 ± 0.72 | 130% |
| citric acid 2000 mg/L | 5.45 ± 2.09 | ▲11% | 4.71 ± 0.15 | 128% |
| citric acid 2000 mg/L+CNSL 100 mg/L | 3.16 ± 0.51 | ▲48% | 4.66 ± 0.24 | 127% |
| citric acid 500 mg/L+DL-malic acid 500 mg/L | 5.43 ± 1.10 | ▲11% | 4.55 ± 0.34 | 124% |
| citric acid500 mg/L+DL-malic acid 500 mg/L+CNSL 100 mg/L | 4.83 ± 1.61 | ▲21% | 4.32 ± 0.39 | 117% |

## Claims

1. An agent for inhibiting methane production for ruminants, comprising as active components:
cashew nut shell liquid, heated cashew nut shell liquid, an anacardic acid, a cardanol, and/or a cardol; and
an organic acid,
wherein the mass ratio of the amount of the cashew nut shell liquid, heated cashew nut shell liquid, anacardic acid, cardanol, and/or cardol, to the amount the organic acid is from 1:5 to 1:20.

2. The agent for inhibiting methane production of claim 1, wherein the organic acid is a C₃₋₁₂ carboxylic acid.

3. The agent for inhibiting methane production of claim 1, wherein the organic acid is a C₃₋₁₀ hydroxycarboxylic acid.

4. The agent for inhibiting methane production of claim 1, wherein the organic acid is citric acid or malic acid.

5. The agent for inhibiting methane production of claim 1, wherein the ruminant is cattle.

6. A food for inhibiting methane production for ruminant, comprising the agent for inhibiting methane production of any one of claims 1 to 5.

7. A method of inhibiting methane production in a ruminant, comprising a step of administering cashew nut shell liquid, heated cashew nut shell liquid, an anacardic acid, a cardanol, and/or a cardol, and an organic acid to the ruminant,
wherein the mass ratio of the daily dose per ruminant of the cashew nut shell liquid, heated cashew nut shell liquid, anacardic acid, cardanol, and/or cardol to the daily dose per ruminant of the organic acid is from 1:5 to 1:20.

8. The method of claim 7, wherein the organic acid is a C₃₋₁₂ carboxylic acid.

9. The method of claim 7, wherein the organic acid is a C₃₋₁₀ hydroxycarboxylic acid.

10. The method of claim 7, wherein the organic acid is citric acid or malic acid.

11. The method of claim 7, wherein the ruminant is cattle.

12. The method of claim 7, wherein the cashew nut shell liquid, heated cashew nut shell liquid, anacardic acid, cardanol, and/or cardol, and the organic acid are pre-mixed and administered.

13. The method of claim 7, wherein the cashew nut shell liquid, heated cashew nut shell liquid, anacardic acid, cardanol, and/or cardol, and the organic acid are separately administered.
